# EUROPÄISCHE PATENTSCHRIFT

(11) **EP 1 057 473 B1**
(45) Veröffentlichungstag und Bekanntmachung des Hinweises auf die Patenterteilung: **15.01.2003**
(21) Anmeldenummer: 99110455.5
(22) Anmeldetag: 29.05.1999
(51) Int. Cl.: A61K 7/38

(54) **Feinteilige Aluminium und Zirkonium enthaltende Antiperspirant Suspensionen mit verbesserter Wirksamkeit und ein Verfahren zu ihrer Herstellung**
Finely particulated aluminium and zirconium containing antiperspirant suspensions with improved activity and process for their preparation
Suspensions anti-transpirantes, à activité améliorée, contenant de fines particules d'un composé d'aluminium et de zirconium, et leur procédé de préparation

(43) Veröffentlichungstag der Anmeldung: 06.12.2000
(73) Patentinhaber: BK Giulini Chemie GmbH & Co. OHG, 67029 Ludwigshafen (DE)
(72) Erfinder: Breker, Johannes, Dr., 67067 Ludwigshafen (DE); Kaufmann, Bruno, 67227 Frankenthal (DE); Reibel, Wolfgang, Dr., 67063 Ludwigshafen (DE); Schanz, Klaus, Dr., 67125 Dannstadt-Schauernheim (DE)

(56) Entgegenhaltungen:
- EP-A- 0 485 012
- WO-A-91/04009
- GB-A- 2 096 891

## Beschreibung

Gegenstand der vorliegenden Erfindung sind feinteilige Aluminium und Zirkonium enthaltende Komplexe in Form einer Suspension, die besonders gute Schweißreduktion auf der menschlichen Haut bewirken und ein Verfahren zu ihrer Herstellung.

Im Stand der Technik sind bereits eine Reihe von Patenten zur Herstellung von Aluminium und Zirkonium Komplexen, die als Antiperspirant-Wirkstoff verwendet werden können, bekannt

In US-2,814,584 und US-2,814,585 (Daley) werden zum ersten Mal Aluminium/Zirkonium/Puffersubstanz Komplexe beschrieben, in denen Harnstoff bzw. Glycin als Pufferkomponente wirkt. Diese Komplexe werden im Falle von Glycin auch ZAG genannt. Ausgegangen wurde von Aluminiumchlorhydrat (ACH) und ZrOCl₂ Lösungen. In US-2,854,382 (Grad) wird darüber hinaus die Möglichkeit beschrieben, als Zr-Quelle anstelle von ZrOCl₂ eine Lösung von ZrO(OH)Cl einzusetzen. Obwohl ein weiter Bereich von Molverhältnissen Al : Zr möglich gewesen wäre, wurde von Daley nur ein Verhältnis von 1,5 : 1 bis 3,5 : 1 beansprucht. Bei Grad darf das Molverhältnis zwischen 0,5 : 1 und 3,0 : 1 liegen In US-2,906,688 beschreiben Beekman et al. ein weiteres Verfahren zur Herstellung von stabilen Aluminium/Zirkonium Komplexen, das dadurch gekennzeichnet ist, daß man eine wässrige Mischung enthaltend ZrOCl₂ x 8 H₂O und Aluminiumhydroxychlorid (ACH) oder Aluminiumtrichlorid / Al Metall erhitzt . Man erhält nicht-gelierende stabile Lösungen mit einem Al / Zr Molverhältnis von 2 bis 8 und einem pH-Wert von über 3.
Callaghan und Phipps beschreiben in ihrem Patent GB-2,144,992 die Herstellung von sog. aktivierten Aluminium/Zirkonium Komplexen (ZAG's), in dem sie wässrige Mischungen enthaltend ZrO(OH)Cl, ACH und Glycin mit einem Al / Zr / Glycin Verhältnis von 4 : 1 : 4 bei 50 °C erhitzten und dabei einen ZAG Komplex enthielten. Die Zusammensetzung eines solchen Komplexes wurde angegeben mit der Formel: Al₂(OH)_{6-y}X_{y})ₐ (ZrO(OH)ₓCl₂₋ₓ)_{b} Neutrale Aminosäure. Der Begriff "aktiviert" wird durch eine neu eingeführte Analysenmethode untermauert. Dabei handelt es sich um die in der Polymerchemie übliche Methode zur Bestimmung der Molekulargewichtsverteilung durch Größenausschlußchromatographie. Die so bestimmbaren Molekulargewichtsverteilungen ( Polymerspeziesverteilungen ) unterscheiden sich bei den Komplexen nach Callaghan und Phipps von denen der zuvor genannten Verfahren und deuten in den Augen der Erfinder auf die chemische Verschiedenheit der neuen Komplexe hin.
Eine Reihe von weiteren Patenten (US-4,775,528, US-5,114,705, US-5,298,640 und US-5,486,347) wurden von Callaghan et al. veröffentlicht, in denen zusätzliche Verfahrensparameter zur Aufheizung der wäßrigen ZrO(OH)Cl, ACH und Glycin Mischungen wie z. B die separate Aufheizung der ACH Lösung zur Verschiebung der Polymerspeziesverteilung ("Aktivierung"), sowie zur Trocknung der Reaktionslösungen enthalten sind.
Die Überlegung, daß die Polymerspeziesverteilung der Komplexe für die Wirksamkeit als Antiperspirant wichtig sein könnte, wurde von Rosenberg et al. im Patent AU 68983/94 auch auf die als Edukt eingesetzte Zirkonium Komponente übertragen. Das von Ihm beschriebene Verfahren ist charakterisiert durch folgende Schritte:
Zunächst wird eine wässrige Mischung aus einem Zirkoniumsalz und Glycin mit einer bestimmten Polymerspeziesverteilung hergestellt. Anschließend wird eine aktivierte ACH Lösung (AACH) hergestellt und mit der Zirkoniumlösung umgesetzt. Diese Lösung wurde sofort durch Sprühtrocknung getrocknet.

In der nachfolgenden Tabelle wird eine Übersicht zu den ZAG Komplexen, die in der Kosmetikindustrie zugelassen sind, gegeben. Die Tabelle wurde von der FDA im Jahr 1982 herausgegeben als Tentative Final Monograph for Antiperspirant Drug Products for .

**Tabelle 1:**

| **Komplexe ohne Glycin** | | | |
|---|---|---|---|
| **Name** | **Me / Cl Verhältnis** | **Al / Zr Verhältnis** | **Glycin** |
| **Aluminium zirkonium Trichlorohydrat (Al/Zr-3)** | 2,1 bis 1,5 | 2 bis 6 | Kein |
| **Aluminium zirkonium Tetrachlorohydrat (Al/Zr-4)** | 1,5 bis 0,9 | 2 bis 6 | Kein |
| **Aluminium zirkonium Pentachlorohydrat (Al/Zr-5)** | 2,1 bis 1,5 | 6 bis 10 | Kein |
| **Aluminium zirkonium Octachlorohydrat (Al/Zr-8)** | 1,5 bis 0,9 | 6 bis 10 | Kein |

**Tabelle 2.**

| **Komplexe enthaltend Glycin als Puffersubstanz:** | | | |
|---|---|---|---|
| **Name** | **Me / Cl Verhältnis** | **Al / Zr Verhältnis** | **Glycin** |
| **Aluminium zirkonium Trichlorohydrex complex (ZAG-3)** | 2,1 bis 1,5 | 2 bis 6 | Variabel |
| **Aluminium zirkonium Tetrachlorohydrex complex (ZAG-4)** | 1,5 bis 0.9 | 2 bis 6 | Variabel |
| **Aluminium zirkonium Pentachlorohydrex complex (ZAG-5)** | 2,1 bis 1,5 | 6 bis 10 | Variabel |
| **Aluminium zirkonium Octachlorohydrex complex (ZAG-8)** | 1,5 bis 0,9 | 6 bis 10 | Variabel |

Es ist bekannt, daß die Aluminiumchlorohydrat-Komplexe (ACH) eine polymere Struktur aufweisen. Diese Verbindungen sind nicht besonders effektiv, d.h. sie zeigen nur eine geringe Schweißreduktion. Sie können jedoch durch Wärme oder chemische Zusätze dahingehend verändert werden, daß eine teilweise Depolymerisation der hochpolymeren Spezies stattfindet. So behandelte Aluminiumchlorohydrat-Komplexe zeigen eine erhöhte Effektivität. Diesen Depolymerisationgrad ("Aktivierungsgrad") kann man mit Hilfe der Größenausschlußchromatographie ( HPLC ) verfolgen. Die Anwesenheit bestimmter Banden im HPLC-Spektrum liefert nach bekannter Lehrmeinung einen Hinweis darauf, ob diese Verbindungen besonders gute schweißhemmende Eigenschaften haben. In diesem Zusammenhang hat sich die Anwesenheit der sog. Bande 3 ( oder Kd = 0,4-0,5 ) als besonders wichtig erwiesen.
Ist die Bande 3 groß, so nahm man an, daß diese Verbindungen besonders effektiv waren.
In der Folgezeit wurde auch das Verhältnis der Bande 2 zu Bande 3 als wichtiges Kriterium für die Beurteilung der Effektivität angesehen.
Bei der Bestimmung des Aktivierungsgrades bzw. der Schweißhemmung von Al/Zr-Verbindungen versuchte man die Erfahrungen und die Methoden aus dem Gebiet der AACH Verbindungen zu übertragen. Auch Al/Zr- Verbindungen zeigen in einem HPLC Chromatogramm eine charakteristische Bandenverteilung.
Einige Forscher benutzen auch Raman- und IR - Spektroskopie, um die Bindungsverhältnisse und die Effektivität dieser Komplexe angeben zu können.
Neben diesen physikalischen Methoden, ist die beste Methode den Aktivierungsgrad und die Effektivität zu ermitteln, die in vitro Methode, der sog. "hot-room" - Test, von dem verschiedene Ausführungsformen beschrieben sind ( A.J. Parisse, in Cosmetic science and Technology Series, Vol. 8 "Clinical Safety and Efficacy Testing of Cosmetics" p.163-223 ). Käuflich erhältliche aktivierte Al/Zr-Komplexe (Antiperspirant-Pulver), zeigen zwar eine bessere Wirksamkeit als die Zr- freien ACH Typen, die erreichbaren Schweißreduktionswerte liegen jedoch für die hohen Ansprüche der Verbraucher zu niedrig. Es bestand also das Bedürfnis nach effektiveren Typen. Es bestand auch ein Bedürfnis nach stabilen Al/Zr - Antiperspirant Wirkstoffen in einer flüssigen, jedoch nicht wässrigen Form.
Bei der Verarbeitung der bekannten pulverförmigen, aktivierten Al/Zr - Wirkstoffe traten Probleme wegen der Neigung der feinstteiligen Pulver zu starker Staubbildung auf. Bei der Weiterverarbeitung der Pulver zu kosmetischen Formulierungen ist deshalb die Einhaltung der Staubgrenzwerte ein wichtiger Punkt um die Gefährdung der Gesundheit der damit beschäftigten Arbeiter auszuschließen.
Es war also bisher nicht möglich, mit den zur Verfügung stehenden Komplexen alle Wünsche der Kosmetikindustrie zu erfüllen.

Es wurde überraschenderweise gefunden, daß man besonders effektive Aluminium und Zirkonium haltige Antiperspirant-Wirkstoffe erhält, wenn diese Wirkstoffe die folgende Formel und Bedingung erfüllen:

Al ₐ(OH)_{(3-b)} X_{b}^{·}(ZrO)_{c}(OH)_{(2-d)}X_{d}^{·}(Aminosäure)ₑ

mit X = Halogen, insbesondere Chlorid,
und a/c = 2,0 bis 10,0
(a + c)/(b + d) = 0,9 bis 2,1
e/c = 0 bis 2,0,
wobei mindestens. 60 % des Zirkoniumgehaltes nach Auflösen in ca. 0,1 n HCl mit EDTA bei pH 0,8 direkt titrierbar ist.
Diese Antiperspirant-Wirkstoffe stellen nicht wässrige Suspensionen dar, die dadurch gekennzeichnet sind, daß die nicht wässrige Phase aus einer im wesentlichen unpolaren, nicht mit Wasser mischbaren organischen Flüssigkeit der Substanzgruppen Alkane, Isoalkane, monofunktionelle Alkohole, polyfunktionelle Alkohole, Fettsäureester von mono- und dibasischen Carbonsäuren mit monofunktionellen und polyfunktionellen Alkoholen, Polyoxyethylenen, Polyoxypropylenen, Polyalkoxylatethern von Alkoholen, cyclischen Silkonen, offenkettigen Silikonen und Kombinationen davon besteht. Insbesondere besteht die nicht wässrige Ölphase aus Silikonöl.

Als Silikonölkomponente werden erfindungsgemäß cyclische Silikone, offenkettige Silikone oder Mischungen davon eingesetzt.

Die erfindungsgemäßen, feinteiligen Antiperspirant - Suspensionen enthalten als Aminosäure Glycin und/oder Alanin.
Die analytische Bestimmung des Zirkonium-Gehalts in einem Zr - haltigen wasserlöslichen Salz wird in der Literatur beschrieben in Fresenius' Zeitschrift für Analytische Chemie, Vol. 246, Seite 391, 1969 oder auch in US-Pharmacopoe XXIII.
Demnach muß der Al /Zr-Komplex längere Zeit in starker Säure gekocht werden (Aufschluß). So erreicht man, daß die Hydrolyse des Zirkoniums zurückgedrängt wird, denn sonst würde man zuwenig Zirkonium finden ( siehe auch Zitat in der Fresenius' Zeitschrift).
Es hat sich gezeigt, daß es möglich ist, zur Abgrenzung der erfindungsgemäßen Wirkstoffe von den Standardkomplexen, zusätzlich ein modifiziertes Analysenverfahren zu verwenden, das sich von den oben geschilderten Standardverfahren unterscheidet.
Dieses modifizierte Verfahren ist durch folgende Schritte charakterisiert:
Es wird eine kleine Menge (1 g) der erfindungsgemäßen Suspension in einem Becherglas mit 50 ml dest. Wasser vermischt. Danach wird mit einigen Tropfen 10 % iger Salzsäure der pH-Wert dieser Mischung auf 0,8 eingestellt und 10 Minuten gerührt. Dabei geht die Al /Zr Verbindung in die wässrige Phase über, die Ölphase trennt sich ab.
Danach wird die EDTA - Lösung zugegeben (20 ml, 0,05 N), und nach Aufheizen auf exakt 50 °C mit dem Indikator versetzt und vor dem Abkühlen auf 40 °C der Überschuß an EDTA z.B. mit einer eingestellten 0,05 n ZrOCl₂ Lösung ( Umschlagspunkt von gelb nach violett bzw. orange - rot ) zurück titriert.

Das Verfahren zur Herstellung von feinteiligen Antiperspirant Suspensionen ist dadurch gekennzeichnet, daß ein als Antiperspirant wirksames Aluminiumsalz mit einem als Antiperspirant wirksamen Zirkoniumsalz, gegebenenfalls in Gegenwart der Aminosäure, bevorzugt Glycin, unter Ausschluß von Feuchtigkeit in einer nicht wässrigen Ölphase gemischt und anschließend vermahlen wird.

Im erfindungsgemäßen Verfahren als ein Antiperspirant wirksames Aluminiumsalz einsetzbar, ist ein basisches Aluminiumhalogenid der folgenden Zusammensetzung :

Al(OH)_{(3-b)}X_{b}

mit X = Halogen, insbesondere Chlorid,
und b = 0,4 bis 3, bevorzugt mit b = 0,45 bis 1,0

In Gegenwart einer Aminosäure, wird ein als Antiperspirant wirksames Aluminiumsalz der folgenden Zusammensetzung eingesetzt:

Al (OH)_{(3-b)} X_{b} (Aminosäure)

mit X = Halogen, insbesondere Chlorid,
b = 0,4 bis 3, bevorzugt mit b = 0,45 bis 1,0
und dem Molverhältnis Aminosäure zu Aluminium von 0 bis 1,0
Besonders bevorzugt sind Aluminium Komplexe der oben genannten Zusammensetzungen, wenn während ihrer Herstellung ein Aktivierungsschritt durchlaufen wurde.

Im erfindungsgemäßen Verfahren als ein Antiperspirant wirksames Zirkoniumsalz einsetzbar, ist ein basisches Zirkoniumhalogenid der folgenden Zusammensetzung:

ZrO (OH)_{(2-d)}X_{d}

mit X = Halogen, insbesondere Chlorid,
und d = 0,5 bis 2, bevorzugt mit d = 0,8 bis 2

In Gegenwart der Aminosäure, z.B. von Glycin, lautet die Formel:

ZrO (OH)_{(2-d)}X_{d} (Glycin)

mit X = Halogen, insbesondere Chlorid,
d = 0,5 bis 2, bevorzugt mit d = 0,8 bis 2
und dem Molverhältnis Aminosäure zu Zr von 0 bis 2,0

Die Mahlung der Antiperspirant Suspension beim erfindungsgemäßen Verfahren ist dadurch gekennzeichnet, daß dieser Vorgang bei Temperaturen unterhalb von 60°C, insbesondere unterhalb von 40°C durchgeführt wird. Die Antiperspirant - Suspensionen werden mit Vorteil in kosmetischen Formulierungen, z. B. in sog. soft - solids oder roll-on's eingesetzt.

Die nachfolgenden Beispiele verdeutlichen die Erfindung näher:

### Beispiele für Al-Zr-Glycin-Suspensionen

### Beispiel 1

In einem 150 l Reaktor mit Propellerrührwerk werden 53,8 kg Cyclomethicone ( DC 345, von der Fa.Dow-Corning) vorgelegt. Unter Rühren werden die folgenden Komponenten eingetragen:
a) 26,68 kg eines aktivierten Aluminiumchlorhydrat Pulvers mit einem Aluminium - Gehalt von 26,0% und einem Chlorid Gehalt von 17,0%, hergestellt nach Pat. US 4,359,456.
b) 20,5 kg eines getrockneten ZrOCl₂ mit einem Zirkonium - Gehalt von 35,7% und einem Chlorid - Gehalt von 27,0% , hergestellt durch Trocknen von kommerziell erhältlichem ZrOCl₂^{·}8 H₂O (Fa. Magnesium Elektron Inc.) Vakuum bei 70° - 80°C.
c) 6,62 kg Glycin

Die homogene Suspension wurde danach mit einer Kugelmühle (Fa. Fryma - CoBall-Mill ) auf eine Endfeinheit von 99,9% < 30µm und 90% < 11µm sehr fein vermahlen. Die dabei erhaltene thixotrope Suspension ist stabil gegenüber Sedimentation. Die Suspension hat einen Zirkoniumgehalt von 6,82 %.

Bestimmt man den Zirkoniumgehalt ohne einen vorherigen Aufschluß mit konzentrierter Säure durch Auflösen des Aluminium-Zirkonium-Glycinates in ca. 0,1 n HCl und anschließender Titration bei pH=0,8 ( Komplexierung mit 0,05 n EDTA bei 50°C /Rücktitration mit 0.05 n ZrOCl₂ Lösung.) so findet man einen Zirkonium - Gehalt von 5,42%. Das entspricht 79% des Gesamt Zirkonium - Gehaltes.

Aufgrund der Zusammensetzung ergeben sich aus der Analyse die Molverhältnisse Al/Zr = 3,2 und (Al + Zr)/Cl = 1,2, so daß nach der FDA - Nomenklatur (FDA -OTC Monograph for Antiperspirants) ein Aluminium / Zirkonium - Tetrachloro -Hydrat - Glycinat vorliegt.

### Beispiel 2

In einem 50 1 Reaktor mit Propellerrührwerk werden 12 kg Cyclomethicone ( DC 345, von der Fa.Dow-Corning) vorgelegt. Unter Rühren wird eine Pulvermischung der beiden folgenden Komponenten eingetragen:
a) 9,6 kg eines aktivierten Aluminiumchlorhydrat Pulvers mit einem Aluminium - Gehalt von 25,7% und einem Chlorid-Gehalt von 17,1%, hergestellt nach US- 4.359.456 und
b) 2,4 kg eines getrockneten ZrOCl₂ mit einem Zirkonium - Gehalt von 35,7% und einem Chlorid - Gehalt von 30,4%, hergestellt durch Trocknen von kommerziell erhältlichem ZrOCl₂^{·}8 H₂O (Fa. Magnesium Elektron Inc.) im Vakuum bei 70° - 80°C.
Die homogene Suspension wurde danach mit einer Kugelmühle (Fa. Fryma - CoBall-Mill ) auf eine Endfeinheit von 99,9% < 30µm und 95% < 10µm sehr fein vermahlen. Die dabei erhaltene thixotrope Suspension ist stabil gegenüber Sedimentation. Die Suspension hat einen Zirkoniumgehalt von 4,06%.

Bestimmt man den Zirkoniumgehalt ohne einen vorherigen Aufschluß mit konzentrierter Säure durch Auflösen des Al-Zr-Chlorhydrats in ca. 0,1 n HCl und anschließender Titration bei pH=0,8 ( Komplexierung mit 0,05 n EDTA bei 50°C / Rücktitration mit 0,05 n ZrOCl2 Lösung.) so findet man einen Zirkonium - Gehalt von 3,08%. Das entspricht 76 % des gesamten Zirkonium Gehaltes.

Aufgrund der Zusammensetzung ergeben sich aus der Analyse die Molverhältnisse Al/Zr = 9,7 und (Al + Zr)/Cl = 1,51, so daß nach der FDA - Nomenklatur (FDA -OTC Monograph for Antiperspirants) ein Aluminium / Zirkonium Pentachlorohydrat vorliegt.

### Beispiel 3

In einer Labor - Fliehkraftkugelmühle werden 30,2 g Cyclomethicone ( DC 345, Fa. Dow Corning) vorgelegt und die 3 folgenden Pulver Komponenten eingetragen:
a) 16,5 g eines aktivierten Aluminiumchlorhydrat Pulver mit einem Aluminium-Gehalt von 25,7% und einem Chlorid-Gehalt von 17,1%, hergestellt nach Pat. US 4.359.456 und
b) 9,6 g eines getrockneten ZrO(OH)CI mit einem Zirkonium - Gehalt von 44,9 % und einem Chlorid - Gehalt von 19,1 % , hergestellt durch Gefriertrocknen einer Zirkonylmonochlorid Lösung.
c) 3,9 g Glycin

Die Mischung wurde danach zur Homogenisierung mit der Labor-Fliehkraftkugelmühle auf eine Endfeinheit von 95% < 15µm sehr fein vermahlen. Die dabei erhaltene thixotrope Suspension ist sedimentationsstabil. Die Suspension hat einen Zirkoniumgehalt von 7,21 %.

Bestimmt man den Zirkoniumgehalt ohne einen vorherigen Aufschluß mit konzentrierter Säure durch Auflösen des Al / Zr - Glycinats in ca. 0,1 n HCl und anschließender Titration bei pH=0,8 (Komplexierung mit 0,05 n EDTA bei 50°C / Rücktitration mit 0,05 n ZrOCl₂ Lösung), so findet man einen Zirkoniumgehalt von 5,03 %. Das entspricht 70 % des gesamten Zirkonium Gehaltes.

Aufgrund der Zusammensetzung ergeben sich aus der Analyse die Molverhältnisse Al/Zr = 3,9 und (Al + Zr)/Cl = 1,51 so daß nach der FDA - Nomenklatur (FDA -OTC Monograph for Antiperspirants) ein Aluminium Zirkonium Trichlorohydrat- Glycinat vorliegt.

### Beispiel 4

In einer Labor-Fliehkraftkugelmühle werden 29,9 g Cyclomethicone ( DC 345, Dow Corning) vorgelegt und die 3 folgenden Komponenten als Pulver eingetragen:
a) 20,4 g eines aktivierten Aluminiumchlorhydrat Pulvers mit einem Aluminium-Gehalt von 25,7% und einem Chlorid-Gehalt von 17,1%, hergestellt nach US- 4.359.456
b) 7,2 g eines getrockneten ZrOCl₂ mit einem Zirkoniumgehalt von 34,9 % und einem Chloridgehalt von 27,3 %, hergestellt durch Trocknen von kommerziell erhältlichem ZrOCl₂^{·}8 H₂O (Fa. Magnesium Elektron Inc.) im Vakuum bei 80°C.
c) 2,52 g Glycin

Die Mischung wurde danach zur Homogenisierung mit der Labor-Fliehkraftkugelmühle auf eine Endfeinheit von 95% < 15µm sehr fein vermahlen. Die dabei erhaltene thixotrope Suspension ist sedimentationsstabil. Die Suspension hat einen Zirkoniumgehalt von 4,36 %.

Bestimmt man den Zirkoniumgehalt ohne einen vorherigen Aufschluß mit konzentrierter Säure durch Auflösen des Al-Zr-Glycinats in ca. 0,1 n HCl und anschließender Titration bei pH=0,8 (Komplexierung mit 0,05 n EDTA bei 50°C / Rücktitration mit 0,05 n ZrOCl₂ Lösung.) so findet man einen Zirkoniumgehalt von 3,07 %. Das entspricht 70,4 % des Zirkoniumgehaltes.

Aufgrund der Zusammensetzung ergeben sich aus der Analyse die Molverhältnisse Al/Zr = 7,05 und (Al + Zr)/Cl = 1,44 so daß nach der FDA - Nomenklatur (FDA -OTC Monograph for Antiperspirants) ein Aluminium / Zirkonium Octachlorohydrat Glycinat vorliegt.

### Vergleichsbeispiel A

In einer Labor-Fliehkraftkugelmühle werden 30 g Cyclomethicone ( DC 345, Dow Coming) vorgelegt und darin 30 g eines kommerziell erhältlichen aktivierten Aluminium Zirkonium Tetrachlorohydrat Glycinats (erhältlich von der Fa. Westwood als Westchlor ZR 35B DM) eingerührt. Die Mischung wurde anschließend zur Homogenisierung mit der Labor-Fliehkraftkugelmühle auf eine Endfeinheit von 95% < 15µm feinst vermahlen. Die dabei erhaltene dünnflüssige Suspension ist nicht sedimentationsstabil. Die Suspension hat einen Zirkoniumgehalt von 5,09 %.

Bestimmt man den Zirkoniumgehalt ohne einen vorherigen Aufschluß mit konzentrierter Säure durch Auflösen des Al-Zr-Glycinats in ca. 0,1 n HCl und anschließender Titration bei pH=0,8 (Komplexierung mit 0,05 n EDTA bei 50°C / Rücktitration mit 0,05 n ZrOCl₂ Lösung.) so findet man einen Zirkoniumgehalt von 0,76 %. Das entspricht 15 % des gesamten Zirkoniumgehaltes.

Aufgrund der Zusammensetzung ergeben sich aus der Analyse die Molverhältnisse Al/Zr = 3,54 und (Al + Zr)/Cl = 1,07, so daß nach der FDA-Nomenklatur (FDA -OTC Monograph for Antiperspirants) ein Aluminium / Zirkonium Tetrachlorohydrat Glycinat vorliegt.

### Vergleichsbeispiel B

Kommerziell erhältliche Aluminium Zirkonium Pentachlorohydrat Lösung ( Zirkonal 50 der Fa. BK Giulini ) wird auf einem Sprühturm bei einer Eintrittstemperatur von 320°C, und einer Austrittstemperatur von 105°C getrocknet. 30 g des sprühgetrockneten Pulvers werden zusammen mit 30 g Cyclomethicone ( DC 345, Fa. Dow - Corning) vermischt.
Die Mischung wird danach zur Homogenisierung mit der Labor-Fliehkraftkugelmühle auf eine Endfeinheit von 95% < 15µm feinst vermahlen. Die dabei erhaltene dünnflüssige Suspension ist nicht sedimentationsstabil. Die Suspension hat einen Zirkoniumgehalt von 4,9 %.

Bestimmt man den Zirkoniumgehalt ohne einen vorherigen Aufschluß mit konzentrierter Säure durch Auflösen des Al-Zr-Chlorhydrats in ca. 0,1 n HCI und anschließender Titration bei pH=0,8 (Komplexierung mit 0,05 n EDTA bei 50°C / Rücktitration mit 0,05 n ZrOCl₂ Lösung.) so findet man einen Zirkoniumgehalt von 0,7 %. Das entspricht 14 % des Zirkoniumgehaltes.

Aufgrund der Zusammensetzung ergeben sich aus der Analyse die Molverhältnisse Al/Zr = 7,04 und (Al + Zr)/Cl = 1,64, so daß nach der FDA-Nomenklatur (FDA -OTC Monograph for Antiperspirants) ein Aluminium Zirkonium Pentachlorohydrat vorliegt.

### Vergleichsbeispiel C

In einer Labor-Fliehkraftkugelmühle werden 30 g Cyclomethicone ( DC 345, Dow Corning) vorgelegt und darin 30 g eines kommerziell erhältlichen aktivierten Aluminium / Zirkonium Trichlorohydrat Glycinats eingerührt.
Die Mischung wurde danach zur Homogenisierung mit der Labor-Fliehkraftkugelmühle auf eine Endfeinheit von 95% < 15µm feinst vermahlen. Die dabei erhaltene dünnflüssige Suspension ist nicht sedimentationsstabil. Die Suspension hat einen Zirkoniumgehalt von 7,40 %.
Bestimmt man den Zirkoniumgehalt ohne einen vorherigen Aufschluß mit konzentrierter Säure durch Auflösen des Al-Zr-Glycinats in ca. 0,1 n HCl und anschließender Titration bei pH=0,8 (Komplexierung mit 0,05 n EDTA bei 50°C / Rücktitration mit 0,05 n ZrOCl₂ Lösung.) so findet man einen Zirkoniumgehalt von 1,4 %. Das entspricht 19 % des gesamten Zirkoniumgehaltes.

Aufgrund der Zusammensetzung ergeben sich aus der Analyse die Molverhältnisse Al/Zr = 3,4 und (Al + Zr)/Cl = 1,51, so daß nach der FDA - Nomenklatur (FDA -OTC Monograph for Antiperspirants) ein Aluminium Zirkonium Trichlorohydrat Glycinat vorliegt.

### Testmethode zur Bestimmung der Wirksamkeit

Die gebräuchlichsten Methoden zur Bestimmung der Wirksamkeit von Antiperspirantien beruhen auf der gravimetrischen Erfassung der Schweißmenge von Probanden, die einem Hitzestreß ausgesetzt wurden.

Die Wirksamkeit des erfindungsgemäßen Produktes aus Beispiel 1 wurde im Vergleich zur Probe aus Vergleichsbeispiel A sowie einer Standard ACH Probe bei der Firma *BioSkin* Institut für Dermatologische Forschung und Entwicklung GmbH, Hamburg geprüft.
Das dort angewandte Verfahren benutzt als Testareal den Rücken der Probanden auf dem mehrere Testfelder (5 cm x 4 cm) zur Verfügung stehen. Dadurch können kontralateral unterschiedliche Prüfproben gegen Placebo oder unbehandeltes Feld geprüft werden.
Mit Silikonöl (DC345, Dow Coming) wurde eine einheitliche Feststoffkonzentration von 22% bei den Proben eingestellt. Die Produkte wurden über 3 Tage bei 16 weiblichen Probanden appliziert. Zur Anpassung an die semiokklusiven Bedingungen in der Achsel wurde nach jeder Applikation von ca. 3,5 mg/cm² der Prüfprodukte eine kontrollierte Okklusion der Testfelder über 150 Minuten vorgenommen.
Das Schwitzen wurde am 4 Tag durch thermische Stimulation in der Sauna erzielt. Der Schweiß wurde von okklusiv aufgeklebten Zellulose pads aufgenommen und anschließend ausgewogen.
Die statische Auswertung der Meßergebnisse ergab, daß die erfindungsgemäße Probe aus Beispiel 1 gegenüber der Vergleichsprobe A eine um 22% höhere Schweißreduktion bewirkt. Gegenüber Standard ACH führt die Vergleichsprobe A zu einer um 25% höheren Schweißreduktion während die erfindungsgemäße Probe aus Beispiel 1 zu einer um 53% besseren Schweißreduktion führt.

## Patentansprüche

1. Feinteilige Suspension in nicht wässriger Phase mit verbesserter Effektivität, die als Wirkstoff einen basischen Aluminium/Zirkonium-Halogenohydrat, gegebenenfalls mit Aminosaüre, Komplex enthält, **dadurch gekennzeichnet daß** min. 60 % des Zirkoniumgehaltes nach Auflösen in 0,1 n HCl mit EDTA bei pH 0,8 direkt titrierbar ist.

2. Feinteilige Antiperspirant - Suspension nach Anspruch 1, **dadurch gekennzeichnet, daß** die nicht wässrige Phase aus einem Öl, bevorzugt aus cyclischen Silikonen, offenkettigen Silikonen oder ihren Mischungen besteht.

3. Feinteilige Antiperspirant - Suspension nach den Ansprüchen 1 bis 2, **dadurch gekennzeichnet, daß** der Wirkstoff als Aminosäure Glycin enthält.

4. Feinteilige Antiperspirant - Suspension nach den Ansprüchen 1 bis 3, **dadurch gekennzeichnet, daß** der Wirkstoff ein basisches Aluminium-Zirkonium-Chlorid ist.

5. Feinteilige Antiperspirant - Suspension nach den Ansprüchen 1 bis 4, **dadurch gekennzeichnet, daß** die Atomverhältnisse der basischen Aluminium-Zirkonium-Chloride den folgenden Bedingungen genügen:
Al : Zr = 2,0 bis 10,0 und (Al + Zr)Cl = 0,9 bis 2,1

6. Feinteilige Antiperspirant - Suspension nach den Ansprüchen 1 bis 5, **dadurch gekennzeichnet, daß** die Atomverhältnisse des basischen Aluminium/Zirkonium-Halogenohydrat-(Aminosäure) Komplexes den folgenden Bedingungen genügen:
Al : Zr = 2,0 bis 10,0 und ( Al + Zr )./ Cl = 0,9 bis 2,1
Aminosäure (Glycin) : Zr = 0,5 bis 2,0

7. Verfahren zur Herstellung von feinteiligen Antiperspirant Suspensionen nach den Ansprüchen 1 bis 6, **dadurch gekennzeichnet, daß** ein als Antiperspirant wirksames Aluminiumsalz mit einem als Antiperspirant wirksamen Zirkoniumsalz unter Ausschluß von Feuchtigkeit in einer nicht wässrigen Ölphase gemischt und anschließend vermahlen wird.

8. Verfahren zur Herstellung von Antiperspirant Suspensionen nach den Ansprüchen 1 bis 7, **dadurch gekennzeichnet, daß** ein als Antiperspirant wirksames Aluminiumsalz mit einem als Antiperspirant wirksamen Zirkoniumsalz in Gegenwart der Aminosäure, bevorzugt Glycin, unter Ausschluß von Feuchtigkeit in einer nicht wässrigen Ölphase gemischt und anschließend vermahlen wird.

9. Verfahren zur Herstellung von Antiperspirant Suspensionen nach den Ansprüchen 1 bis 8, **dadurch gekennzeichnet, daß** als ein Antiperspirant wirksames Aluminiumsalz ein basisches Aluminiumchlorid der folgenden Zusammensetzung eingesetzt wird:
Al (OH)₍₃₋ₓ₎ Cl ₓ
mit x = 0,4 bis 3, bevorzugt mit x = 0,45 bis 1,0

10. Verfahren zur Herstellung von Antiperspirant Suspensionen nach den Ansprüchen 1 bis 9, **dadurch gekennzeichnet, daß** als ein Antiperspirant wirksames Aluminiumsalz ein basisches Aluminiumchlorid der folgenden Zusammensetzung eingesetzt wird:
Al (OH)₍₃₋ₓ₎ Cl ₓ
mit x = 0,4 bis 3, bevorzugt mit x = 0,5 bis 1,0, wobei das als Antiperspirant wirksame Aluminiumsalz die Aminosäure Glycin enthält.

11. Verfahren zur Herstellung von Antiperspirant Suspensionen nach den Ansprüchen 1 bis 10, **dadurch gekennzeichnet, daß** als ein Antiperspirant wirksames Zirkoniumsalz ein basisches Zirkoniumchlorid der folgenden Zusammensetzung eingesetzt wird:
Zr O (OH)_{(2-y)} Cl_{y}
mit y = 0,5 bis 2, bevorzugt mit y = 0,9 bis 2

12. Verfahren zur Herstellung von Antiperspirant Suspensionen nach den Ansprüchen 1 bis 11, **dadurch gekennzeichnet, daß** als ein Antiperspirant wirksames Zirkoniumsalz ein basisches Zirkoniumchlorid der folgenden Zusammensetzung eingesetzt wird:
Zr O (OH)_{(2-y)}Cl _{y}
mit y = 0,5 bis 2, bevorzugt mit y = 0,9 bis 2, wobei das als Antiperspirant wirksame Zirkoniumsalz die Aminosäure Glycin enthält.

13. Verfahren zur Herstellung von Antiperspirant Suspensionen nach Anspruch 1 bis 12, **dadurch gekennzeichnet, daß** die Mahlung bei Temperaturen unterhalb von 50°C, insbesondere unterhalb von 30°C durchgeführt wird.

14. Kosmetische Antiperspirant Formulierung, **dadurch gekennzeichnet, daß** sie als Wirkkomponente die feinteilige Antiperspirant Suspension nach einem der Ansprüche 1 bis 6 enthält.

## Claims

1. Finely divided suspension in non-aqueous phase with improved effectiveness, containing as the active agent a basic aluminum/zirconium halogen hydrate complex, possibly with amino acids, **characterized in that** at least 60% of the zirconium content may be directly titrated after solution in 0.1 n HCl with EDTA at a pH of 0.8.

2. Finely divided antiperspirant suspension according to Claim 1, **characterized in that** the non-aqueous phase comprises an oil, preferably made of cyclic silicones, open-chain silicones or mixtures thereof.

3. Finely divided antiperspirant suspension according to the claims 1 to 2, **characterized in that** the active agent contains glycine as the amino acid.

4. Finely divided antiperspirant suspension according to the claims 1 to 3, **characterized in that** the active agent is a basic aluminum zirconium chloride.

5. Finely divided antiperspirant suspension according to the claims 1 to 4, **characterized in that** the atomic ratios of the basic aluminum zirconium chlorides fulfil the following conditions:
Al:Zr = 2.0 to 10.0 and (Al + Zr):Cl = 0.9 to 2.1.

6. Finely divided antiperspirant suspension according to the claims 1 to 5, **characterized in that** the atomic ratios of the basic aluminum/zirconium-halogen hydrate-(amino acid) complex fulfil the following conditions:
Al:Zr = 2.0 to 10.0 and (Al + Zr):Cl = 0.9 to 2.1
Amino acid (glycine):Zr = 0.5 to 2.0.

7. Method for manufacturing finely divided antiperspirant suspensions according to the claims 1 to 6, **characterized in that** an aluminum salt effective as an antiperspirant is mixed with a zirconium salt effective as an antiperspirant, while excluding moisture, in a non-aqueous oil phase and subsequently ground.

8. Method for manufacturing antiperspirant suspensions according to the claims 1 to 7, **characterized in that** an aluminum salt effective as an antiperspirant is mixed with a zirconium salt effective as an antiperspirant in the presence of the amino acid, preferably glycine, while excluding moisture, in a non-aqueous oil phase and subsequently ground.

9. Method for manufacturing antiperspirant suspensions according to the claims 1 to 8, **characterized in that** as an aluminum salt effective as an antiperspirant, a basic aluminum chloride with the following composition is used:
Al(OH)₍₃₋ₓ₎Clₓ
where x = 0.4 to 3, and preferably x = 0.45 to 1.0.

10. Method for manufacturing antiperspirant suspensions according to the claims 1 to 9, **characterized in that** as an aluminum salt effective as an antiperspirant, a basic aluminum chloride with the following composition is used:
Al(OH)₍₃₋ₓ₎Clₓ
where x = 0.4 to 3, and preferably x = 0.5 to 1.0, whereby the aluminum salt effective as an antiperspirant contains the amino acid glycine.

11. Method for manufacturing antiperspirant suspensions according to the claims 1 to 10, **characterized in that** as a zirconium salt effective as an antiperspirant a basic zirconium chloride with the following composition is used:
ZrO(OH)_{(2-y)}Cl_{y}
where y = 0.5 to 2, and preferably y = 0.9 to 2.

12. Method for manufacturing antiperspirant suspensions according to the claims 1 to 11, **characterized in that** as a zirconium salt effective as an antiperspirant, a basic zirconium chloride with the following composition is used:
ZrO(OH)_{(2-y)}Cl_{y}
where y = 0.5 to 2, preferably y = 0.9 to 2, where the zirconium salt effective as an antiperspirant contains the amino acid glycine.

13. Method for manufacturing antiperspirant suspensions according to the claims 1 to 12, **characterized in that** the grinding is carried out at temperatures below 50°C, and particularly below 30°C.

14. Cosmetic antiperspirant formulation, **characterized in that**, as the active agent it contains the finely divided antiperspirant suspension according to one of the claims 1 to 6.

## Revendications

1. Suspension à particules fines en phase non aqueuse à efficacité améliorée, contenant comme principe actif un complexe basique d'hydrate halogéné aluminium/zirconium, avec des acides aminés le cas échéant, **caractérisée en ce qu'**au moins 60 % de la teneur en zirconium est directement titrable après dissolution dans 0,1 n HCI avec EDTA à un pH de 0,8.

2. Suspension anti-transpirante à particules fines selon revendication 1, **caractérisée en ce que** la phase non aqueuse consiste en une huile, préférentiellement de silicones cycliques, silicones à chaîne ouverte ou leurs composés.

3. Suspension anti-transpirante à particules fines selon les revendications 1 à 2, **caractérisée en ce que** le principe actif contient de la glycine comme acide aminé.

4. Suspension anti-transpirante à particules fines selon les revendications 1 à 3, **caractérisée en ce que** le principe actif est un chlorure basique d'aluminium-zirconium.

5. Suspension anti-transpirante à particules fines selon les revendications 1 à 4, **caractérisée en ce que** les rapports atomiques des chlorures basiques d'aluminium-zirconium satisfont aux conditions suivantes :
Al : Zr = 2,0 à 10,0 et (Al + Zr)CI = 0,9 à 2,1

6. Suspension anti-transpirante à particules fines selon les revendications 1 à 5, **caractérisée en ce que** les rapports atomiques du complexe basique d'hydrate halogéné (aminoacide) aluminium/zirconium satisfont aux conditions suivantes :
Al : Zr = 2,0 à 10,0 et ( Al + Zr )./Cl = 0,9 à 2,1
Acide aminé (glycine) : Zr = 0,5 à 2,0

7. Procédé pour la fabrication de suspensions anti-transpirantes à particules fines selon les revendications 1 à 6, **caractérisé en ce qu'**un sel d'aluminium efficace comme anti-transpirant est mélangé avec un sel de zirconium efficace comme anti-transpirant en excluant toute humidité dans une phase d'huile non aqueuse, avant d'être pulvérisé.

8. Procédé pour la fabrication de suspensions anti-transpirantes selon les revendications 1 à 7, **caractérisé en ce qu'**un sel d'aluminium efficace comme anti-transpirant est mélangé avec un sel de zirconium efficace comme anti-transpirant en présence de l'acide aminé, préférentiellement de la glycine, en excluant toute humidité dans une phase d'huile non aqueuse, avant d'être pulvérisé.

9. Procédé pour la fabrication de suspensions anti-transpirantes selon les revendications 1 à 8, **caractérisé en ce qu'**un chlorure d'aluminium basique de la composition suivante est utilisé en tant que d'aluminium efficace comme anti-transpirant :
Al(OH)₍₃₋ₓ₎Clₓ
avec x = 0,4 à 3, préférentiellement avec x = 0,45 à 1,0.

10. Procédé pour la fabrication de suspensions anti-transpirantes selon les revendications 1 à 9, **caractérisé en ce qu'**un chlorure d'aluminium basique de la composition suivante est utilisé en tant que d'aluminium efficace comme anti-transpirant :
Al (OH)₍₃₋ₓ₎ Cl ₓ
avec x = 0,4 à 3, préférentiellement avec x = 0,5 à 1,0, le sel d'aluminium efficace comme anti-transpirant contenant l'acide aminé glycine.

11. Procédé pour la fabrication de suspensions anti-transpirantes selon les revendications 1 à 10, **caractérisé en ce qu'**un chlorure de zirconium basique de la composition suivante est utilisé en tant que sel de zirconium efficace comme anti-transpirant :
Zr O (OH)_{(2-y)}Cl_{y}
avec y = 0,5 à 2, préférentiellement avec y = 0,9 à 2.

12. Procédé pour la fabrication de suspensions anti-transpirantes selon les revendications 1 à 11, **caractérisé en ce qu'**un chlorure de zirconium basique de la composition suivante est utilisé en tant que de zirconium efficace comme anti-transpirant :
Zr O (OH)_{(2-y)} Cl_{y}
avec y = 0,5 à 2, préférentiellement avec y = 0,9 à 2, le sel de zirconium efficace comme anti-transpirant contenant l'acide aminé glycine.

13. Procédé pour la fabrication de suspensions anti-transpirantes selon les revendications 1 à 12, **caractérisé en ce que** la pulvérisation est effectuée à des températures inférieures à 50°C, en particulier inférieures à 30°C.

14. Formule cosmétique d'anti-transpirant, **caractérisée en ce qu'**elle contient comme composant actif la suspension anti-transpirante à particules fines selon l'une des revendications 1 à 6.
